(19) Europäisches Patentamt / European Patent Office / Office européen des brevets

(11) **EP 1 874 941 B1**

(12) **FASCICULE DE BREVET EUROPEEN**

(45) Date de publication et mention de la délivrance du brevet:
**07.09.2016 Bulletin 2016/36**

(21) Numéro de dépôt: **06755458.4**

(22) Date de dépôt: **27.04.2006**

(51) Int Cl.:
*C12P 3/00* (2006.01)          *C02F 1/52* (2006.01)
*C02F 3/34* (2006.01)          *C02F 9/00* (2006.01)
*C02F 101/10* (2006.01)          *C02F 101/20* (2006.01)

(86) Numéro de dépôt international:
**PCT/FR2006/000954**

(87) Numéro de publication internationale:
**WO 2006/117458 (09.11.2006 Gazette 2006/45)**

(54) **PROCEDE DE PRODUCTION D'ACIDE SULFHYDRIQUE ET SON APPLICATION, NOTAMMENT A LA DEPOLLUTION D'EFFLUENTS CONTENANT DES METAUX LOURDS**

VERFAHREN ZUR HERSTELLUNG VON WASSERSTOFFSULFID UND DESSEN VERWENDUNG, IM BESONDEREN ZUR ENTGIFTUNG VON SCHWERMETALLHALTIGEN STRÖMEN

METHOD FOR PRODUCING HYDROGEN SULPHIDE AND THE USE THEREOF, IN PARTICULAR, FOR DEPOLLUTING HEAVY METAL-CONTAINING FLOWS

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **29.04.2005 FR 0504386**

(43) Date de publication de la demande:
**09.01.2008 Bulletin 2008/02**

(73) Titulaire: **Institut de Recherche pour le Développement ( IRD)**
**75480 Paris Cedex 10 (FR)**

(72) Inventeurs:
• **OLLIVIER, Bernard, Marcel, Nöel**
  **F-13360 Roquevaire (FR)**
• **COMBET-BLANC, Yannick Aman B.,**
  **Rés. Les Sources**
  **F-13009 Marseille (FR)**
• **FARDEAU, Marie-Laure**
  **F-13170 Les Pennes-Mirabeau (FR)**
• **CHARDIN, Bruno,**
  **Résidence Campagne Valérie**
  **F-13400 Aubagne (FR)**

(74) Mandataire: **Grosset-Fournier, Chantal Catherine et al**
**Grosset-Fournier & Demachy**
**54, rue Saint-Lazare**
**75009 Paris (FR)**

(56) Documents cités:
**US-A- 5 587 079**

• **PIKUTA E V ET AL: "Desulfonatronum thiodismutans sp. nov., a novel alkaliphilic, sulfate-reducing bacterium capable of lithoautotrophic growth" INTERNATIONAL JOURNAL OF SYSTEMATIC AND EVOLUTIONARY MICROBIOLOGY 2003 UNITED KINGDOM, vol. 53, no. 5, 2003, pages 1327-1332, XP002359059 ISSN: 1466-5026**
• **ZHILINA TATJANA N ET AL: "Desulfonatronum cooperativum sp nov., a novel hydrogenotrophic, alkaliphilic, sulfate-reducing bacterium, from a syntrophic culture growing on acetate" INTERNATIONAL JOURNAL OF SYSTEMATIC AND EVOLUTIONARY MICROBIOLOGY, vol. 55, no. Part 3, mai 2005 (2005-05), pages 1001-1006, XP002359060 ISSN: 1466-5026**

EP 1 874 941 B1

**Description**

L'invention concerne un procédé de production d'acide sulfhydrique et son application, notamment à la dépollution d'effluents contenant des métaux lourds.

Les métaux lourds peuvent se révéler fortement toxiques pour l'homme et son environnement. Ces métaux lourds ne sont pas biodégradables, si bien qu'ils présentent un caractère cumulatif. Ainsi, des normes de rejet de plus en plus restrictives ont été imposées aux activités industrielles rejetant des métaux.

Les procédés les plus couramment utilisés pour séparer les métaux lourds contenus dans les effluents industriels recourent à la formation d'hydroxydes métalliques. Cependant, ces procédés ne donnent pas toujours satisfaction au regard des normes environnementales actuelles concernant les taux de métaux dissous acceptables dans les effluents.

Une autre technologie déjà mise en oeuvre implique la formation de sulfures métalliques., Celle-ci suppose l'utilisation de polysulfures de synthèse coûteux ou la formation d'hydrogène sulfuré gazeux par l'action de l'acide chlorhydrique sur le sulfure de sodium.

Des alternatives biologiques à ce mode de traitement, impliquant encore une production d'hydrogène sulfuré gazeux, ont été proposées par le passé. Ces alternatives biologiques reposent sur deux types principaux de mécanismes biologiques susceptibles de conduire à la formation d'hydrogène sulfuré :

1) La réduction assimilatrice du sulfate est une fonction anabolique qui permet à la plupart des bactéries, des champignons ou des plantes d'incorporer le soufre dans les acides aminés (cystéine, méthionine et cystine), les vitamines (thiamine et biotine) et autres molécules soufrées (ferrédoxine par exemple...) présentes dans les cellules de ces organismes. Cette réduction n'aboutit jamais directement à la production d'hydrogène sulfuré. Néanmoins, ce dernier est libéré de façon indirecte lors de la fermentation de la matière organique protéique.

2) La réduction dissimilatrice des sulfates est effectuée par les bactéries sulfato-réductrices. Dans ce processus respiratoire anaérobie, le sulfate est utilisé comme accepteur terminal d'électrons lors de l'oxydation de l'hydrogène ou de composés organiques réduits tels que l'acétate et le propionate. Au cours de ce métabolisme, les substrats sont le plus souvent partiellement oxydés en acétate ou, dans quelques cas, totalement oxydés pour conduire à la formation de $CO_2$.

De nombreux brevets ou demandes de brevets concernent l'utilisation de bactéries sulfato-réductrices pour la précipitation d'ions métalliques en sulfures métalliques, afin de dépolluer des effluents tels que des effluents miniers ou des eaux usées.

Ainsi, les documents WO 80/02281 et US 4,522,723, concernent l'utilisation de bactéries de type *Desulfovibrio* ou *Desulfotomaculum* afin de réduire les taux de métaux lourds dans des effluents. Le document US 4,108,722 envisage l'injection de bactéries *Vibrio* et *Desulfovibrio* dans des réservoirs aquifères souterrains contaminés. Le document US 5,062,956 concerne plus spécifiquement le traitement du chrome hexavalent. Trois autres documents, US 5,587,079, WO 97/29055 et WO 02/06540 proposent d'autres procédés de précipitation biologique des métaux, avec la particularité que la précipitation se fait séquentiellement, en faisant varier le pH (principalement entre 2,5 et 6,5) afin de précipiter par exemple d'abord le cuivre, puis le zinc etc... On peut également citer le document WO 97/05237, qui divulgue l'utilisation de bactéries méthylotrophes, c'est à dire capables d'utiliser le méthanol comme unique source de carbone. Il faut encore noter que des systèmes de co-culture de bactéries de souches différentes ont également été envisagés, par exemple dans le document US 4,789,478 ou le document EP 0 692 458.

Un inconvénient des procédés précédemment cités réside dans le fait que des quantités parfois importantes d'hydrogène sulfuré sous forme gazeuse ($H_2S$) sont produites au cours de ces procédés. C'est notamment le cas lorsqu'on souhaite obtenir une précipitation maximale dés métaux initialement dissous dans l'effluent à dépolluer, ce qui suppose de faire intervenir de l'hydrogène sulfuré en excès. Or, l'hydrogène sulfuré sous forme gazeuse est toxique, corrosif, nocif pour l'environnement, et exige un traitement approprié supplémentaire contraignant.

Un moyen d'éviter cet inconvénient consiste à produire de l'acide sulfhydrique dissous (sous forme HS⁻) à la place de l'hydrogène sulfuré gazeux. Pour ce faire, il est souhaitable que le pH de la solution de culture dans laquelle est produit le sulfure par les bactéries sulfato-réductrices soit aussi élevé (basique) que possible. Or l'utilisation d'un pH élevé, chez la grande majorité des bactéries sulfato-réductrices, nuit au rendement de la production de sulfure, et peut même être létal pour la culture.

L'un des aspects de l'invention est de produire de l'acide sulfhydrique, essentiellement soluble, avec un bon rendement, à l'aide de bactéries sulfato- ou thiosulfato-réductrices.

L'un des autres aspects de l'invention est de proposer de nouvelles conditions de culture de certaines bactéries sulfato-réductrices, permettant notamment de mettre en oeuvre leurs propriétés thiosulfato-réductrices.

L'un des autres aspects de l'invention est de proposer un procédé de dépollution d'effluents contenant des métaux lourds à l'aide d'acide sulfhydrique produit par la culture de bactéries sulfato ou thiosulfato-réductrices.

Un autre aspect encore de l'invention est de produire de l'acide sulfhydrique pour dépolluer des effluents contenant des métaux lourds, en évitant la présence indésirable d'hydrogène sulfuré gazeux.

Ces différents aspects sont obtenus en utilisant certaines bactéries sulfato-réductrices alcalophiles récemment découvertes, non utilisées à ce jour pour produire de l'acide sulfhydrique ou a fortiori pour dépolluer des effluents chargés en métaux.

La présente invention est illustrée par l'utilisation de bactéries alcalophiles sulfato-réductrices ou thiosulfato-réductrices choisies parmi au moins une espèce de la famille des *Desulfohalobiaceae* ou du genre *Desulfonatronum* ou dont le gène codant pour l'ARN ribosomique 16 S présente une homologie d'au moins 97 % avec le gène correspondant de l'une quelconque des espèces de la famille des *Desulfohalobiaceae* ou du genre *Desulfonatronum,* pour produire de l'acide sulfhydrique sous forme majoritairement soluble dans un milieu aqueux.

Par « bactéries alcalophiles » on entend des bactéries dont la vie, la croissance, les diverses activités métaboliques et enzymatiques, s'effectuent de façon préférentielle à un pH basique.

Une description taxonomique, morphologique et physiologique des bactéries utilisées dans l'invention a été donnée dans les articles suivants :

- Pikuta et al., Desulfonatronum lacustre gen. nov. sp. nov.: a new alkaliphilic sulfate-reducing bacterium utilizing ethanol ; Microbiology 67, 105 ;
- Zhilina et al., Desulfonatronovibrio hydrogenovorans gen. nov. sp. nov., an alkaliphilic, sulfate-reducing bacterium ; Int. J. Syst. Bacteriol. Jan. 1997, p. 144 ;
- Pikuta et al., Desulfonatronum thiodismutans sp. nov., a novel alkaliphilic, sulfate-reducing bacterium capable of lithoautotrophic growth ; Int. J. Syst. Evol. Microbiol. 53, 1327.

On désigne par « homologie » la proportion d'identité entre deux séquences d'acides nucléiques. Cette homologie peut être mesurée en cherchant à aligner lesdites séquences en utilisant un algorithme tel que défini dans Altschul et al. (Nucl. Acid Res. 25:3389, 1997) ou en utilisant par exemple le logiciel Clustal W, bien connu de l'homme du métier et décrit dans Thompson et al. (Nucl. Acid Res. 22:4673, 1994).

Par « forme majoritairement soluble », on entend un rapport d'acide sulfhydrique soluble produit sur l'hydrogène sulfuré gazeux produit supérieur à 1, et en particulier supérieur à 100.

Comme cela sera détaillé ci-après, on utilise de préférence, pour les conditions de culture des bactéries sulfato-réductrices selon l'invention, des substrats originaux, à savoir le formiate et le thiosulfate. Le formiate sert de source d'énergie, tandis que le thiosulfate sert d'accepteur d'électrons et de source de soufre : les souches de bactéries utilisées dans l'invention sont donc thiosulfato-réductrices en plus d'être sulfato-réductrices. Cette propriété de certaines bactéries sulfato-réductrices à utiliser le thiosulfate comme substrat n'a pas été mise en oeuvre dans les procédés de dépollution des métaux précédemment cités.

L'utilisation des bactéries alcalophiles sulfato ou thiosulfato-réductrices dans les conditions de l'invention permet de produire de l'acide sulfhydrique sous forme très majoritairement soluble, et avec un rendement exceptionnellement élevé.

Un autre avantage décisif de l'invention est la possibilité qu'elle offre de travailler avec une culture pure sans devoir effectuer de stérilisation, ce qui permet des économies notables. En effet, les conditions particulières de culture qui sont employées (pH élevé, minéralité, absence d'$O_2$, concentration élevée d'acide sulfhydrique...) garantissent une forte pression de sélection.

L'utilisation des bactéries alcalophiles sulfato-réductrices ou thiosulfato-réductrices selon l'invention s'effectue à un pH supérieur ou égal à environ 9, notamment à un pH supérieur ou égal à environ 9,5, notamment à un pH supérieur où égal à environ 10.

La proportion de l'hydrogène sulfuré produit selon l'invention qui est sous forme gazeuse (c'est à dire le rapport du sulfure d'hydrogène gazeux produit sur l'acide sulfhydrique solubilisé produit) dépend en effet du pH auquel est produit l'hydrogène sulfuré. A un pH acide, la forme prédominante du sulfure d'hydrogène est la forme gazeuse. A un pH de 7 il y a environ autant de molécules d'$H_2S$ que d'ions $HS^-$. A un pH de 9, la proportion d'hydrogène sulfuré gazeux est de seulement environ 1/100. A un pH de 9,5, la proportion d'hydrogène sulfuré gazeux est de seulement environ 1/500. A un pH de 10, la proportion d'hydrogène sulfuré gazeux est de seulement environ 1/1000,

Dans une illustration de l'invention, l'utilisation des bactéries alcalophiles sulfato-réductrices ou thiosulfato-réductrices s'effectue sous forme de culture en présence d'un composé organique servant de donneur d'électrons, notamment le formiate, et en présence d'un composé soufré servant d'accepteur d'électrons, notamment le thiosulfate.

Toutefois, il importe de noter que, selon les souches bactériennes utilisées, d'autres substrats peuvent servir à la production de sulfure : par exemple le sulfate, le sulfite ou le soufre en tant qu'accepteur d'électrons, et l'éthanol ou le dihydrogène en tant que donneur d'électrons.

Quoi qu'il en soit, la production d'acide sulfhydrique s'effectue dans l'invention par l'intermédiaire de la réduction d'un composé soufré. Dans le cas où ce composé soufré est le thiosulfate ($S_2O_3^{2-}$), les mécanismes enzymatiques de réduction impliqués reposent notamment sur la thiosulfate soufre transférase (ou rhodanèse) et sur la thiosulfate réductase.

Dans le premier cas, le bilan de la réaction de réduction du thiosulfate équivaut à une oxydation d'une fonction thiol par le thiosulfate, dont la partie sulfonyle est réduite en sulfite :

$$S_2O_3^{2-} + 2\ RS^- + H^+ \rightarrow SO_3^{2-} + R\text{-}SS\text{-}R + HS^-$$

Dans le second cas, le thiosulfate est clivé en sulfite et sulfure, et le sulfite est ensuite réduit en sulfure, selon le bilan final :

$$S_2O_3^{2-} + 4\ H_2 \rightarrow 2\ HS^- + 3\ H_2O$$

L'invention est illustrée par un procédé de production d'acide sulfhydrique sous forme majoritairement soluble en milieu aqueux comportant :

- une étape de culture de bactéries alcalophiles sulfato-réductrices ou thiosulfato-réductrices choisies parmi au moins une espèce de la famille des *Desulfohalobiaceae* ou du genre *Desulfonatronum* ou dont le gène codant pour l'ARN ribosomique 16 S présente une homologie d'au moins 97 % avec, le gène correspondant de l'une quelconque des espèces de la famille des *Desulfohalobiaceae* ou du genre *Desulfonatronum,* en présence d'un composé organique servant de donneur d'électrons, notamment le formiate, et en présence d'un composé soufré servant d'accepteur d'électrons, notamment le thiosulfate, ce qui conduit à la formation d'acide sulfhydrique.

L'invention concerne donc un procédé de production d'acide sulfhydrique sous forme majoritairement soluble en milieu aqueux comportant

- une étape de culture de bactéries alcalophiles sulfato-réductrices ou thiosulfato-réductrices choisies parmi au moins une espèce de la famille des *Desulfohalobiaceae* ou du genre *Desulfonatronum* ou dont le gène codant pour l'ARN ribosomique 16 S présente une homologie d'au moins 97 % avec le gène correspondant de l'une quelconque des espèces de la famille des *Desulfohalobiaceae* ou du genre *Desulfonatronum,* en présence d'un composé organique servant de donneur d'électrons, notamment le formiate, et en présence d'un composé soufré servant d'accepteur d'électrons, notamment le thiosulfate, ce qui conduit à la formation d'acide sulfhydrique,

ledit procédé comprenant une succession des deux étapes suivantes :

- une première étape dans laquelle la culture des bactéries est effectuée dans des conditions appropriées à la croissance desdites bactéries et à la production concomitante d'acide sulfhydrique, et
- une seconde étape dans laquelle la culture des bactéries est effectuée dans des conditions appropriées à la production d'acide sulfhydrique en l'absence de croissance desdites bactéries,

la succession desdites étapes constituant un cycle pouvant éventuellement être répété plusieurs fois, et la première étape dudit procédé étant effectuée à un pH allant de 9 à 10 et la seconde étape dudit procédé étant effectuée à un pH supérieur à 10.

Cette étape de culture des bactéries peut s'effectuer par exemple dans un réacteur standard tel que ceux disponibles sur le marché pour la fermentation, à l'échelle pilote tout comme à l'échelle industrielle.

Dans le procédé de production d'acide sulfhydrique selon l'invention, la culture des bactéries alcalophiles sulfato-réductrices ou thiosulfato-réductrices est effectuée à un pH supérieur ou égal à environ 9, notamment à un pH supérieur ou égal à environ 9,5, notamment à un pH supérieur ou égal à environ 10.

Au besoin, le pH peut être mesuré et régulé par l'ajout de substances acides et / ou basiques.

Selon un mode de réalisation préféré du procédé de production d'acide sulfhydrique selon l'invention, on choisit les bactéries de telle sorte qu'elles présentent une tolérance à l'acide sulfhydrique, ladite tolérance étant caractérisée en ce que les bactéries tolèrent des concentrations en acide sulfhydrique au moins supérieures à 20 mM.

Par « tolérance » on entend la survie des bactéries et le maintien d'une activité métabolique normale, que l'on peut observer par la consommation de la source d'énergie.

Cette bonne tolérance des bactéries en culture permet d'obtenir des solutions à concentration élevée en acide sulfhydrique.

De manière avantageuse, les bactéries utilisées dans le procédé de production d'acide sulfhydrique selon l'invention appartiennent à l'espèce *Desulfonatronum lacustre.*

De manière avantageuse, les bactéries utilisées dans le procédé de production d'acide sulfhydrique selon l'invention appartiennent à l'espèce *Desulfonatronovibrio hydrogenevorans.*

Selon un mode de réalisation avantageux de l'invention, la culture intervenant dans le procédé de production d'acide sulfhydrique est effectuée sur un support adapté à la croissance des bactéries, conduisant à la formation d'un biofilm.

Par « biofilm », on entend des bactéries préférentiellement fixées sur un support adapté, de type pouzzolane, Cloisonyl®, Bio-Net® ou Sessil® par exemple, au lieu d'une simple suspension de bactéries en solution. La culture en biofilms permet d'obtenir localement des concentrations de cellules bactériennes importantes et de produire plus rapidement de l'acide sulfhydrique. En outre, peu de cellules bactériennes sont ôtées du réacteur lors du prélèvement de la solution de culture contenant l'acide sulfhydrique, les bactéries n'étant pas majoritairement en suspension.

Le procédé de production d'acide sulfhydrique selon l'invention comporte une succession des deux étapes suivantes :

- une première étape dans laquelle la culture des bactéries est effectuée dans des conditions appropriées à la croissance desdites bactéries et à la production concomitante d'acide sulfhydrique, et
- une seconde étape dans laquelle la culture des bactéries est effectuée dans des conditions appropriées à la production d'acide sulfhydrique en l'absence de croissance desdites bactéries,

la succession desdites étapes constituant un cycle pouvant éventuellement être répété plusieurs fois.

Par « croissance desdites bactéries » on entend notamment leur multiplication par division cellulaire.

Par «production d'acide sulfhydrique en l'absence de croissance desdites bactéries », on entend d'une part l'arrêt substantiel ou le ralentissement notable de la croissance, c'est à dire de la multiplication des bactéries, mais également d'autre part la survie de ces bactéries ou au moins le maintien de l'activité enzymatique d'origine bactérienne provoquant la formation d'acide sulfhydrique dans le milieu.

De manière préférée, le passage de l'une à l'autre desdites étapes est effectué notamment au moyen d'ajout d'une ou plusieurs substances chimiques acides ou basiques entraînant une modification du pH du milieu de culture des bactéries.

Dans le procédé de l'invention, ladite première étape est effectuée à un pH allant d'environ 9 à environ 10 et ladite seconde étape est effectuée à un pH supérieur à environ 10.

En effet, les bactéries utilisées dans l'invention sont capables de maintenir leur activité de production d'acide sulfhydrique à des pH alcalins particulièrement extrêmes, c'est à dire notamment à un pH supérieur à environ 10. Il est donc avantageux de cultiver dans un premier temps les bactéries de l'invention à leur pH optimal de culture, qui est en général compris entre environ 9 et environ 10, afin d'obtenir le plus grand nombre possible de bactéries, puis de passer dans un second temps à un pH supérieur à environ 10 afin de continuer à produire de l'acide sulfhydrique, mais dans des conditions chimiques du milieu telles que le rapport du sulfure d'hydrogène gazeux produit sur l'acide sulfhydrique produit est aussi faible que possible, et en particulier inférieur à 1/100.

Selon un mode de réalisation préféré de l'invention, l'acide sulfhydrique est produit à une concentration supérieure ou égale à environ 10 mM, notamment à une concentration supérieure ou égale à environ 20 mM, notamment à une concentration supérieure ou égale à environ 30 mM, notamment à une concentration supérieure ou égale à environ 40 mM.

La vitesse spécifique de production typique d'acide sulfhydrique pouvant être atteinte selon l'invention est d'au moins 2,9 mmol HS$^-$ g$^{-1}$ h$^{-1}$ et peut aller jusqu'à 5 mmol HS$^-$ g$^{-1}$ h$^{-1}$.

Selon un autre mode de réalisation préféré de l'invention, l'acide sulfhydrique est produit par une culture de bactéries appartenant à l'espèce *Desulfonatronum lacustre,* le composé organique servant de donneur d'électrons étant le formiate, le composé soufré servant d'accepteur d'électrons étant le thiosulfate, et les bactéries sont cultivées en continu sur un biofilm à un pH supérieur à environ 10.

L'invention est également illustrée par un procédé de dépollution d'un effluent contenant un ou plusieurs métaux solubilisés comportant :

- une étape de production d'acide sulfhydrique sous forme majoritairement soluble en milieu aqueux au moyen d'une culture de bactéries alcalophiles sulfato-réductrices ou thiosulfato-réductrices choisies parmi au moins une espèce de la famille des *Desulfohalobiaceae* ou du genre *Desulfonatronum* ou dont le gène codant pour l'ARN ribosomique 16 S présente une homologie d'au moins 97 % avec le gène correspondant de l'une quelconque des espèces de la famille des *Desulfohalobiaceae* ou du genre *Desulfonatronum* en présence d'un composé organique servant de donneur d'électrons, notamment le formiate, et en présence d'un composé soufré servant d'accepteur d'électrons, notamment le thiosulfate, et
- une étape de mise en contact dudit effluent avec l'acide sulfhydrique obtenu à l'étape précédente, entraînant la réduction desdits métaux solubilisés et / ou la précipitation desdits métaux solubilisés sous forme de sulfures métalliques, ladite mise en contact s'effectuant à un pH allant d'environ 2 à environ 12.

La présente invention a également pour objet un procédé de dépollution d'un effluent contenant un ou plusieurs métaux solubilisés comportant :

- une étape de production d'acide sulfhydrique sous forme majoritairement soluble en milieu aqueux au moyen d'une culture de bactéries alcalophiles sulfato-réductrices ou thiosulfato-réductrices choisies parmi au moins une espèce de la famille des *Desulfohalobiaceae* ou du genre *Desulfonatronum* ou dont le gène codant pour l'ARN ribosomique 16 S présente une homologie d'au moins 97 % avec le gène correspondant de l'une quelconque des espèces de la famille des *Desulfohalobiaceae* ou du genre *Desulfonatronum* en présence d'un composé organique servant de donneur d'électrons, notamment le formiate, et en présence d'un composé soufré servant d'accepteur d'électrons, notamment le thiosulfate, et
- une étape de mise en contact dudit effluent avec l'acide sulfhydrique obtenu à l'étape précédente, entraînant la réduction desdits métaux solubilisés et / ou la précipitation desdits métaux solubilisés sous forme de sulfures métalliques, ladite mise en contact s'effectuant à un pH allant de 2 à 12,

l'étape de production d'acide sulfhydrique comprenant une succession des deux étapes suivantes :

- une première étape dans laquelle la culture des bactéries est effectuée dans des conditions appropriées à la croissance desdites bactéries et à la production concomitante d'acide sulfhydrique, et
- une seconde étape dans laquelle la culture des bactéries est effectuée dans des conditions appropriées à la production d'acide sulfhydrique en l'absence de croissance desdites bactéries,

la succession desdites étapes constituant un cycle pouvant éventuellement être répété plusieurs fois, et
la première étape dudit procédé étant effectuée à un pH allant de 9 à 10 et la seconde étape dudit procédé étant effectuée à un pH supérieur à 10.

Par « dépollution d'un effluent contenant un ou plusieurs métaux solubilisés » on entend la réduction significative de la concentration en un ou plusieurs métaux dissous dans l'effluent, et notamment une réduction en-dessous des seuils imposés par les diverses normes environnementales de rejets.

Des exemples de seuils de concentrations de métaux dans les rejets liquides actuellement imposés par la législation française sont les suivants : 0,5 mg/L pour le cuivre ; 0,5 à 2 mg/L pour le zinc ; 0,05 à 0,5 mg/L pour l'arsenic ; 0,05 à 0,2 mg/L pour le cadmium ; 0,1 mg/L pour le chrome hexavalent ; 0,5 à 2 mg/L pour l'étain ; 0,03 à 0,05 mg/L pour le mercure ; 0,5 à 2 mg/L pour le nickel ; 0,1 à 0,5 mg/L pour le plomb. Il faut encore noter que les seuils peuvent varier selon les industries concernées, et que des arrêtés préfectoraux fixent parfois localement des seuils jusqu'à 100 fois inférieurs aux valeurs précitées.

Or, à l'exception du cas particulier du chrome, quasiment tous les métaux peuvent être précipités en sulfures métalliques par l'action de l'acide sulfhydrique et présentent une moindre solubilité sous forme de sulfures métalliques que sous forme d'hydroxydes métalliques.

En effet les solubilités minimales observées, à divers pH, pour les hydroxydes et les sulfures métalliques sont les suivantes :

- $5,2 \times 10^{-2}$ mg/L pour le sulfure d'arsenic (et pas de formation d'hydroxyde d'arsenic) ;
- $6,7 \times 10^{-10}$ mg/L pour le sulfure de cadmium contre $2,3 \times 10^{-5}$ mg/L pour l'hydroxyde de cadmium ;
- $1,0 \times 10^{-8}$ mg/L pour le sulfure de cuivre contre $2,2 \times 10^{-2}$ mg/L pour l'hydroxyde de cuivre ;
- $3,8 \times 10^{-8}$ mg/L pour le sulfure d'étain contre $1,1 \times 10^{-4}$ mg/L pour l'hydroxyde d'étain ;
- $2,1 \times 10^{-3}$ mg/L pour le sulfure de manganèse contre 1,2 mg/L pour l'hydroxyde de manganèse ;
- $9,0 \times 10^{-20}$ mg/L pour le sulfure de mercure contre $3,9 \times 10^{-4}$ mg/L pour l'hydroxyde de mercure ;
- $6,9 \times 10^{-8}$ mg/L pour le sulfure de nickel contre $6,9 \times 10^{-3}$ mg/L pour l'hydroxyde de nickel ;
- $3,8 \times 10^{-9}$ mg/L pour le sulfure de plomb contre 2,1 mg/L pour l'hydroxyde de plomb ; et
- $2,3 \times 10^{-7}$ mg/L pour le sulfure de zinc contre 1,1 mg/L pour l'hydroxyde de zinc ;

Dans le cas particulier du chrome sous sa forme hexavalente, la précipitation sous forme de sulfure n'est pas possible, mais en revanche l'acide sulfhydrique permet de réduire l'ion $Cr^{6+}$ en ion $Cr^{3+}$ (Kim et al. 2001, Chromium VI reduction by hydrogen sulfide in aqueous media: stoichiometry and kinetics. Environ. Sci. Technol. 35(11):2219-2225), qui est beaucoup moins toxique et moins soluble, notamment sous sa forme hydroxyde.

De plus, la solubilité des hydroxydes métalliques est généralement minimale pour une certaine valeur de pH optimale, alors que la solubilité des sulfures métalliques est typiquement une fonction purement décroissante du pH, si bien que l'on a toujours intérêt, du seul point de vue de la solubilité des sulfures, à travailler à un pH aussi basique que possible.

En résumé, la dépollution d'effluents selon l'invention est donc particulièrement avantageuse par rapport à des procédés faisant appel à une simple précipitation sous forme d'hydroxydes pour :

- l'arsenic, qui ne peut être insolubilisé en hydroxyde, et dont l'insolubilisation en sulfure permet de respecter les valeurs seuils de rejet ;
- le chrome hexavalent, qui ne peut précipiter efficacement en hydroxyde sans avoir été préalablement réduit en chrome trivalent par l'acide sulfhydrique ; et
- le manganèse, le zinc et le plomb, dont la précipitation en sulfures permet de respecter les valeurs seuils de rejet, à la différence de leur précipitation en hydroxydes.

Des exemples d'effluents pouvant être traités selon l'invention sont : les effluents des industries chimique, parachimique, pétrolière, et notamment de l'industrie du revêtement et de la production de pigments ; les effluents de l'industrie minérale et notamment de la verrerie (fort rejet de plomb) ; les effluents du secteur mécanique et du traitement des surfaces ; les effluents du secteur sidérurgique et métallurgique (notamment pour les rejets d'arsenic, de chrome VI, de plomb et de manganèse) ; les effluents du secteur du traitement des déchets.

Dans ledit procédé de dépollution d'un effluent contenant un ou plusieurs métaux solubilisés selon l'invention, l'étape de production d'acide sulfhydrique peut être effectuée selon l'une quelconque des manières décrites précédemment.

Selon un mode préféré de réalisation dudit procédé de dépollution d'un effluent contenant un ou plusieurs métaux solubilisés, l'étape de mise en contact de l'effluent avec l'acide sulfhydrique est effectuée à un pH neutre ou basique.

Ce mode de réalisation permet de minimiser un éventuel dégagement d'hydrogène sulfuré gazeux lors de l'étape de mise en contact et permet une précipitation plus complète des sulfures métalliques, qui sont moins solubles à un pH élevé qu'à un pH faible.

De manière avantageuse, dans ledit procédé de dépollution d'un effluent contenant un ou plusieurs métaux solubilisés, on effectue la production d'acide sulfhydrique et la mise en contact de l'effluent avec l'acide sulfhydrique dans des cuves distinctes, notamment respectivement une cuve de culture et une cuve de réaction, ledit procédé comportant une étape intermédiaire entre l'étape de production d'acide sulfhydrique et l'étape de mise en contact de l'effluent avec l'acide sulfhydrique, ladite étape intermédiaire consistant en l'injection de tout ou partie de l'acide sulfhydrique produit dans la cuve de culture dans la cuve de réaction.

Au stade de la décontamination, c'est à dire de la mise en contact dans la cuve de réaction entre l'effluent pollué et tout ou partie de l'acide sulfhydrique produit, ou après ce dit stade, il est possible d'ajouter des agents coagulants tels que $FeCl_3$, afin de provoquer la floculation des sulfures métalliques insolubilisés, puis éventuellement de faire passer l'effluent dans un décanteur lamellaire afin de séparer les précipités après sédimentation.

Selon un mode de réalisation particulier du procédé de dépollution d'un effluent contenant un ou plusieurs métaux solubilisés selon l'invention, une fraction de l'effluent est injectée dans la cuve de culture.

Ce mode de réalisation peut être qualifié de production « au contact » ou « au contact partiel » de l'effluent. Dans les cas où la composition de l'effluent est telle que l'effluent ne réduit pas significativement la capacité de production d'acide sulfhydrique par les bactéries lorsque celles-ci sont cultivées au contact de cet effluent, ce mode de réalisation peut présenter un avantage économique.

Selon un autre mode de réalisation du procédé de dépollution d'un effluent contenant un ou plusieurs métaux solubilisés selon l'invention, particulièrement préféré, aucune fraction de l'effluent n'est injectée dans la cuve de culture.

Ce mode de réalisation peut être qualifié de production « en parallèle » de l'effluent. Il est particulièrement avantageux, puisque les bactéries destinées à produire l'acide sulfhydrique n'entrent jamais en contact avec l'effluent pollué ; or une telle mise en contact peut nuire à la capacité de production d'acide sulfhydrique des bactéries dans une proportion difficilement prévisible, selon leur plus ou moins bonne résistance à la présence de métaux dissous dans le milieu de culture.

De manière avantageuse, le procédé de dépollution d'un effluent contenant un ou plusieurs métaux solubilisés selon l'invention est tel que l'étape de mise en contact de l'effluent avec l'acide sulfhydrique dans la cuve de réaction est effectuée en absence d'une phase gazeuse, de telle sorte qu'il n'y a pas de dégagement de sulfure d'hydrogène gazeux.

Par « en absence d'une phase gazeuse » on entend : en l'absence de tout contact avec l'air ou tout autre gaz. Selon la terminologie consacrée, cette mise en contact intervient donc en « milieu noyé ». Cette caractéristique est avantageuse dans la mesure où un dégagement d'hydrogène sulfuré gazeux est à envisager en cas de présence d'une phase gazeuse, et ce d'autant plus que le pH dans la cuve de réaction peut être basique mais aussi neutre ou acide, auquel cas l'équilibre chimique entre acide sulfhydrique solubilisé et hydrogène sulfuré gazeux est déplacé au profit de ce dernier.

Il est à noter que dans les autres modes de réalisation précédemment cités du procédé de dépollution selon l'invention, en cas de présence d'une phase gazeuse, la concentration d'hydrogène sulfuré gazeux éventuellement dégagé lors de l'étape de mise en contact est de préférence inférieure à 5 mg/m$^3$.

En ce qui concerne la cuve de culture, celle-ci peut comporter une phase gazeuse. Dans ce cas, l'hydrogène sulfuré gazeux éventuellement présent dans cette phase gazeuse peut servir à réduire les composés oxydants apportés dans le milieu. En cas de surpression gazeuse, on peut neutraliser l'hydrogène sulfuré gazeux par lavage à la soude (NaOH). En tout état de cause, la concentration d'hydrogène sulfuré gazeux dégagé dans la cuve de culture est de préférence inférieure à 5 mg/m$^3$.

Selon un mode de réalisation préféré de l'invention, le procédé susmentionné de dépollution d'un effluent contenant un ou plusieurs métaux solubilisés comporte les étapes supplémentaires :

- de mesure de la concentration d'acide sulfhydrique produit dans la cuve de culture,
- d'estimation de la concentration du ou des différents métaux solubilisés dans l'effluent devant être dépollué, et
- d'ajustement de la quantité de solution contenant de l'acide sulfhydrique devant être injectée dans la cuve de réaction en fonction du résultat de ladite mesure et de ladite estimation.

En d'autres termes, selon ce mode de réalisation il est possible d'utiliser le procédé de dépollution d'un effluent selon l'invention dans des applications variées, c'est à dire qu'il est possible de dépolluer des effluents présentant des caractéristiques très diverses en termes de concentrations de métaux dissous, par une adaptation simple et immédiate du procédé de l'invention. Il suffit en effet de disposer d'une production suffisamment importante d'acide sulfhydrique, puis d'adapter la quantité d'acide sulfhydrique à mettre en contact avec l'effluent à la nature de cet effluent devant être dépollué.

Des exemples non-limitatifs de métal ou de métaux solubilisés dans un effluent pollué pouvant être traités au moyen de l'invention sont : le cuivre, le zinc, l'arsenic, le cadmium, le chrome, notamment sous sa forme hexavalente, l'étain, le manganèse, le mercure, le nickel, et le plomb.

Une récupération, éventuellement sélective, des précipités métalliques obtenus grâce au procédé selon l'invention, en vue de leur valorisation, est possible.

**Description des figures**

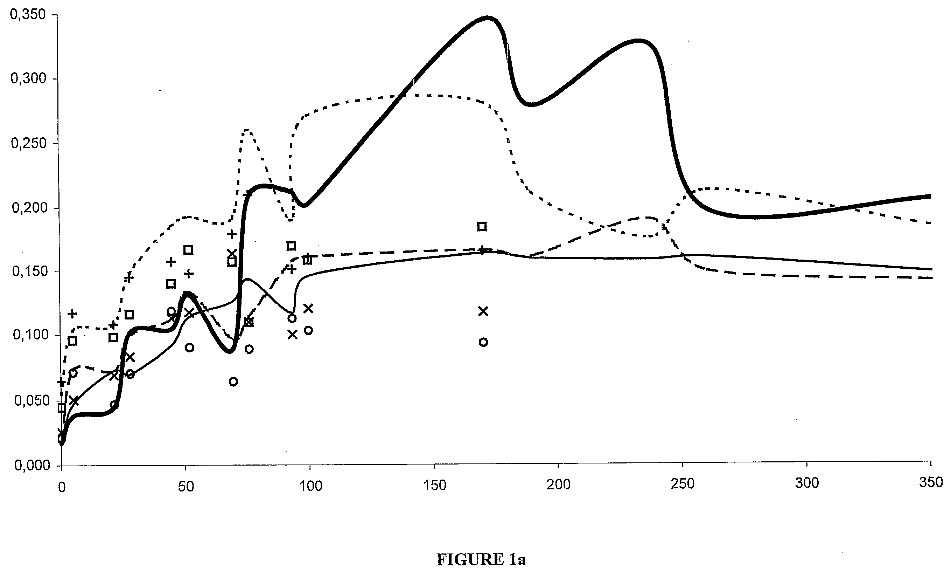

La figure 1a représente la croissance d'une culture de *Desulfonatronum lacustre* dans différentes conditions de culture. L'axe des abscisses indique le temps en heures. L'axe des ordonnées indique la mesure de la densité optique à 580 nm.

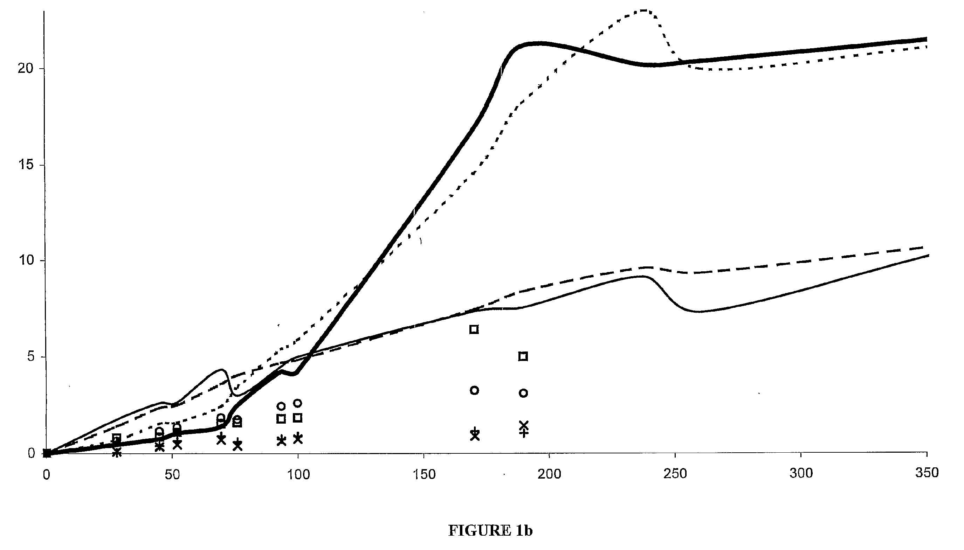

La figure 1b représente la production d'acide sulfhydrique dissous lors d'une culture de *Desulfonatronum lacustre* dans différentes conditions de culture. L'axe des abscisses indique le temps en heures. L'axe des ordonnées indique la concentration de HS$^-$ en mM.

Pour les figures 1a et 1b, les éléments des conditions de culture qui varient sont les suivants : F = présence de formiate ; E = présence d'éthanol ; S = présence de sulfate ; T = présence de thiosulfate ; YE = présence d'extrait de levure. Les différentes séries de conditions sont représentées sur les deux figures de la façon suivante :

- Symboles ✕ : E et T ;
- Symboles ○ : E et S ;
- Symboles + : E, T et YE ;
- Symboles □ : E, S et YE ;
- Trait continu épais : F et T ;
- Trait en pointillés longs : F et S ;
- Trait en pointillés courts : F, T et YE ; et
- Trait continu fin : F, S et YE.

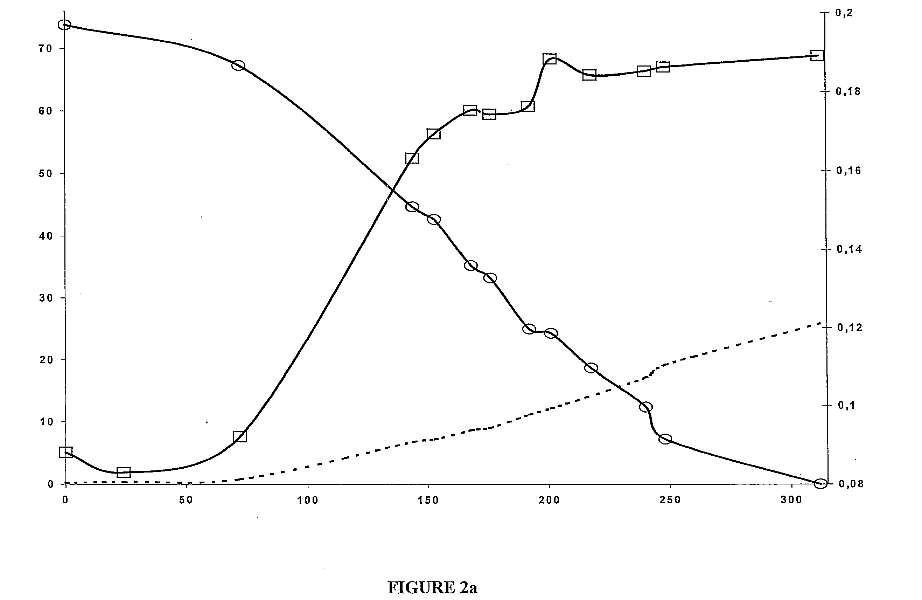

La figure 2a représente le résultat d'une culture de *Desulfonatronum lacustre* en réacteur de type « batch » à pH 9,5. L'évolution de la concentration d'acide sulfhydrique est représentée en trait pointillé ; celle de la concentration de formiate est représentée en trait continu avec symboles ○ ; celle de la densité optique à 580 nm (indicative de la concentration en bactéries) est représentée en trait continu avec symboles □. L'axe des abscisses correspond au temps en heures. L'axe des ordonnées de gauche correspond aux concentrations en mM (pour l'acide sulfhydrique et le formiate) ; et l'axe des ordonnées de droite correspond à la DO à 580 nm.

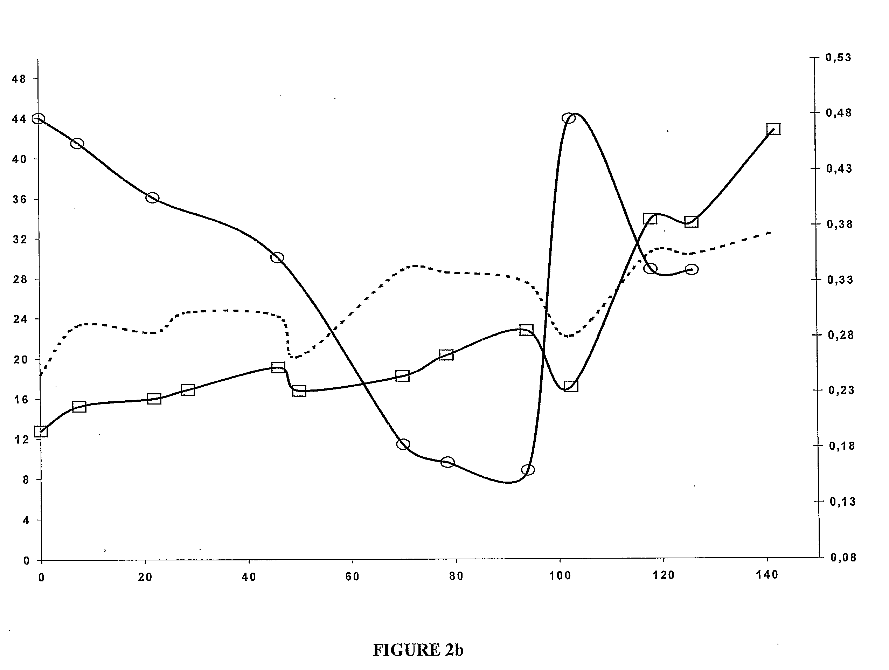

La figure 2b représente le résultat d'une culture de *Desulfonatronum lacustre* en réacteur de type « batch » à pH 10. L'évolution de la concentration d'acide sulfhydrique est représentée en trait pointillé ; celle de la concentration de formiate est représentée en trait continu avec symboles ○ ; celle de la densité optique à 580 nm (indicative de la concentration en bactéries) est représentée en trait continu avec symboles □. L'axe des abscisses correspond au temps en heures. L'axe des ordonnées de gauche correspond aux concentrations en mM (pour l'acide sulfhydrique et le formiate) ; et l'axe des ordonnées de droite correspond à la DO à 580 nm. Il faut noter qu'à t = 92 h on procède à une injection de milieu concentré (voir exemple correspondant ci-dessous).

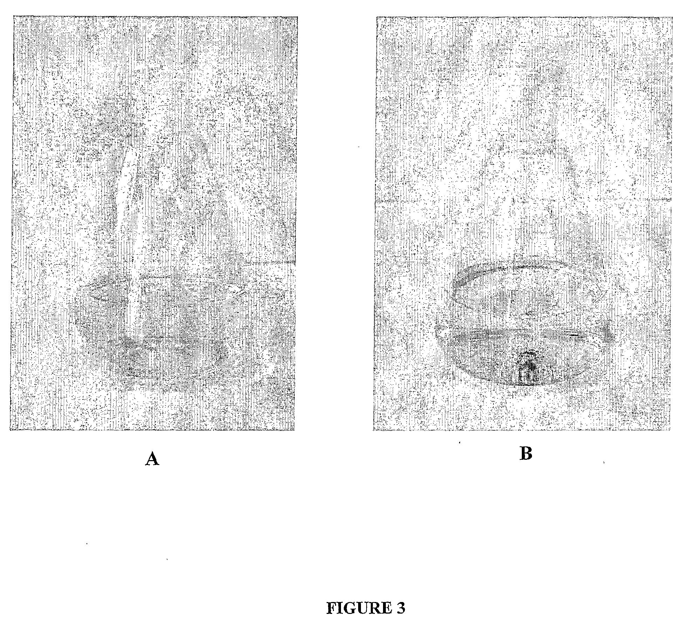

La figure 3 représente le résultat d'une expérience de précipitation de métaux lourds selon l'invention. La photo A représente un échantillon de 150 ml d'effluent industriel contenant essentiellement du plomb solubilisé. La photo B représente le même échantillon immédiatement après injection de 5 ml de milieu de culture obtenu après 60 jours de culture de *Desulfonatronum lacustre,* contenant plus de 50 mM d'acide sulfhydrique. Des particules noires de sulfure de plomb sont visibles.

## Partie expérimentale

### Souches bactériennes et milieux de culture

Deux souches bactériennes sont étudiées ici : *Desulfonatronum lacustre* (pH : 9,5) ; *Desulfonatrovibrio hydrogenevorans* (pH : 9,5). Les deux souches sont cultivées à 37°C. Les sources d'énergie (donneurs d'électron) testées sont l'éthanol et le formiate ; les accepteurs d'électrons testés sont le sulfate et le thiosulfate. Les souches sont détaillées dans les exemples ci-après. La sélection des souches a été réalisée par des cultures de 5 ml dans des tubes de Hungate.

La composition du milieu de culture (dit MLF) est la suivante (en g/L) :

| | |
|---|---|
| $NH_4Cl$ | 1 |
| $K_2HPO_4$ | 0,3 |
| $KH_2PO_4$ | 0,3 |
| $CaCl_2.2H_2O$ | 0,1 |
| KCl | 0,1 |
| $MgCl_2.6H_2O$ | 0,1 |
| Cystéine | 0,5 |
| $Na_2S$ | 0,04 % |
| Oligo-éléments de Widdel | 1 mL |
| Extrait de levure | 0,1 |

Le sulfure de sodium, la source d'énergie, l'accepteur d'électrons et le tampon défini ci-après sont ajoutés au milieu juste avant l'ensemencement de celui-ci. Le sulfure de sodium permet de fortement diminuer le potentiel redox du milieu, créant ainsi des conditions favorables à la croissance des bactéries anaérobies strictes.

Le tampon pour les cultures réalisées à pH 9,5 est le $Na_2CO_3$ 1,6%

### Dispositif de fermentation

Les tests de production d'acide sulfhydrique ont été réalisés par culture en suspension de la souche sélectionnée dans un réacteur de type « batch » (CHEMAP AG, Switzerland) d'une capacité de 20 L.

La régulation du pH, de la température et de la vitesse d'agitation est réalisée au niveau d'un boîtier de commande jouxtant le fermenteur (réacteur). Le pH est régulé en continu au moyen d'une sonde pH plongée dans le milieu de culture. Lorsqu'une valeur de pH inférieure au point de consigne est détectée, un volume de solution de soude ou de carbonate est injecté dans le fermenteur par l'intermédiaire d'une pompe péristaltique.

La température du fermenteur (37°C) est régulée par circulation d'eau thermostatée à travers le corps métallique de l'appareil.

L'agitation (15. tours/minute) est réalisée par une hélice de type marine.

L'arrivée d'azote est régulée par un débitmètre à bille. Une faible surpression est maintenue dans le fermenteur par le flux d'azote entrant et par l'immersion de la sortie des gaz dans une solution de soude 1N. Cette solution de soude permet également de neutraliser les effluents gazeux de $H_2S$ pouvant être émis lors de la fermentation.

Un robinet situé en fond de cuve du réacteur permet de réaliser des prélèvements lors de la fermentation.

Pour son démarrage, le réacteur, contenant 10 litres de milieu de culture MLF et le thiosulfate, est initialement stérilisé à la vapeur puis refroidi sous flux d'azote. Le formiate et un inoculum de 2 litres d'une culture en fin de phase exponentielle de croissance sont ensuite injectés. Le pH est alors ajusté par ajout de carbonate 40 mM stérile et désoxygéné.

Par la suite, les composés ajoutés à la culture (soude, carbonate, milieu MLF) ne sont pas préalablement stérilisés ou désaérés.

## Suivi de la croissance

La croissance des bactéries est suivie à l'aide d'un spectrophotomètre par mesure de la densité optique des cultures à une longueur d'onde de 580 nm.

Des études précédemment menées ont permis d'établir une relation linéaire entre la concentration cellulaire de la bactérie *Thermotoga elfii* (g de poids sec/litre), bactérie thiosulfato-réductrice, et la densité optique à 580 nm lorsque cette dernière ne dépasse pas 0,8 : [Cellules] = $0,73 \times DO_{580nm}$. Cette méthode est donc appliquée ici pour les autres bactéries.

La présence de précipités dans les cultures de *Desulfonatronum lacustre* impose une minéralisation de ceux-ci préalablement à la mesure de la densité optique. Cette minéralisation est réalisée par ajout de 400 μL d'acide persulfurique 1M à 4 mL de culture bactérienne. Après agitation, le mélange est laissé au repos pendant 2 minutes avant lecture de la densité optique à 580 nm.

## Dosage colorimétrique du sulfure

Le dosage du sulfure dissous est réalisé selon la méthode de Cord Ruwish (Cord Ruwish R., J. Microbiol. Methods. 4 : 33-36, 1985) : après prélèvement de 0,1 mL de milieu de culture, celui-ci est rapidement mélangé par vortexage avec 4 mL de $CuSO_4$ 5 mM, HCl 50 mM. Le sulfure de cuivre de couleur rouge bordeaux ainsi formé est dosé par mesure de l'absorption à 480 nm et comparaison avec une courbe étalon pré-enregistrée.

Le dosage du sulfure total est réalisé par alcalinisation préalable du milieu de culture à pH 12. Ceci entraîne la dissolution de la totalité du sulfure qui est ensuite dosé comme indiqué ci-dessus.

## Dosage colorimétrique du thiosulfate

Le dosage du thiosulfate est réalisé selon la méthode décrite par Nor & Tabatabai (Nor Y.M§ and Tabatabai M.A., Anal. Lett. 8 : 537-547, 1975) : 1 mL d'échantillon est mélangé à 1 ml KCN 0,1 M.

$$S_2O_3^{2-} \rightarrow SO_3^{2-} + CNS^-$$

Après 15 minutes, 2 mL $CuCl_2$ 0,33 M puis 1 mL $Fe(NO_3)_3$-$HNO_3$ sont ajoutés.

$$CNS^- + Fe^{3+} \rightarrow Fe\text{-}CNS$$

Le mélange est ajusté à 25 mL avec de l'eau osmosée puis, après agitation et deux minutes d'attente, la quantité de soufre présente dans l'échantillon est déterminée par mesure de l'absorption du complexe ferro-thiocyanique à 460 nm et comparaison avec une courbe étalon pré-enregistrée.

## Dosage du sulfate

Le sulfate est dosé suivant la méthode de Tabatabai (Tabatabai M.A., Sulfur Institut Journal 10 : 11-13, 1974) : 1 mL d'échantillon est prélevé stérilement puis acidifié avec 250 ml de HCl 1N dans un tube Eppendorf afin d'enlever les sulfures. Les cellules sont éliminées par centrifugation (14000 rpm, 3 minutes) et 0,5 mL de surnageant est repris dans 4,5 mL d'eau distillée. L'ajout de 250 μL de $BaCl_2$ (1% p/v) - gélatine (0,3% p/v) permet de précipiter le sulfate sous forme de sulfate de baryum. Après 30 minutes de repos, le mélange est homogénéisé au vortex et l'absorption est mesurée à 420 nm.

L'étalonnage est réalisé suivant le même protocole avec 5 mL d'eau distillée et 250 μL de solution $BaCl_2$-gélatine.

La calibration est effectuée à partir d'étalons de sulfate ($Na_2SO_4$ 1 mM, 5 mM, 10 mM et 20 mM) auxquels on applique le protocole ci-dessus.

## Dosage des sucres, des acides organiques et des alcools

Le dosage des produits solubles du métabolisme est réalisé par séparation par chromatographie haute pression en phase liquide (HLPC) sur colonne ORH801 (Interaction chemicals) avec détection par un réfractomètre différentiel RID 6A (Shimadzu). L'éluant est une solution $H_2SO_4$ 0,005 N filtrée (0,65 μm Millipore).

Les échantillons sont centrifugés (1300 tours.min$^{-1}$ $\times$ 15 min) avant d'être injectés.

Dosage des gaz

Le dosage des produits gazeux du métabolisme bactérien est réalisé par chromatographie en phase gazeuse (CPG). Le chromatographe (Chrompack CP 9000) est équipé de deux colonnes montées en série (Silicagel GC et Molecular Sieve 5A). La détection se fait par conductivité thermique à l'aide d'un catharomètre. Le gaz vecteur est de l'hélium à 2 bars, le courant du filament détecteur est à 70 mA. Les injections de gaz (0,1 mL) sont réalisées à l'aide d'une seringue munie d'une vanne de type Minimert™ permettant de maintenir la pression de l'échantillon.

**Exemples**

Description de *Desulfonatronum lacustre*

Il s'agit d'une bactérie sulfato et thiosulfato-réductrice, alcalophile, halotolérante et chimiolithotrophe.
Morphologie : vibrion, 0,7-0,9 x 2-3 $\mu$m, isolé, en paires ou en chaînes spiralées, mobile par un flagelle polaire. Gram négatif. Multiplication par fission binaire. Colonie dans gélose : lenticulaire, 0,5-2 mm $\varnothing$, jaunâtre puis brune, translucide, à bord entier.

Métabolisme : non fermentative
Accepteurs d'électrons : sulfate, sulfite, thiosulfate.
Dismutation du thiosulfate en sulfure + sulfate.
Donneurs d'électrons : $H_2$-$CO_2$, formate, éthanol $\rightarrow$ acétate.

Pas de croissance sur acétate, propionate, butyrate, pyruvate, lactate, malate, fumarate, succinate, méthanol, glycérol, choline, bétaine, casamino acides, extrait de levure, glucose, fructose, mannose, xylose, rhamnose.
Syntrophie avec *Spirochaeta alcalica* ou *Desulfonatronovibrio hydrogenovorans.* Présence de cyt c, absence de désulfoviridine.
Croissance stimulée par extrait de levure ou acétate.

Physiologie :     T optimum = 37-40°C (20-45°C).
                         pH optimum = 9,5 (8-10).
                         NaCl optimum = 0% w/v (0-10% w/v).

Dépendante des ions sodium et du carbonate.

ADN: 57,3 mol% G+C
Séquence ARN 16S: Y14594
Souche type Z-7951 (DSM 10312).
Origine: sédiment du lac Khadyn.
Référence : Pikuta EV, Zhilina TN, Zavarzin GA, Kostrikina NA, Osipov GA, Rainey FA (1998) Desulfonatronum lacustre gen. nov., sp. nov., a new alkaliphilic sulfate-reducing bacterium utilizing ethanol. Microbiology (Engl. Tr. Mikrobiologyia) 67, 123-131.

Description de *Desulfonatrovibrio hydrogenevorans*

Bactérie sulfato et thiosulfato-réductrice, alcalophile, faiblement halophile.

Morphologie : vibrion, 0,5 x 1,5-2 $\mu$m, 1 flagelle polaire, appendices filamenteux, isolé ou en paires ou en courtes chaînes, Gram négatif.
Métabolisme : lithohétérotrophe
Accepteurs d'électrons : sulfate, sulfite, thiosulfate.
Donneurs d'électrons : $H_2$ + $CO_2$, formate.
Sources de carbone : acétate avec extrait de levure ou vitamines.
Formation de sulfure sur diméthyl sulfoxyde sans croissance.
Croissance inhibée par le soufre.
Disproportionation du thiosulfate en sulfate et sulfure.
Pas de désulfoviridine.
Physiologie : alcalophile faiblement halophile

T optimum: 37°C (15-43°C)

pH optimum : 9,5-9,7 (8-10,2)

NaCl optimum : 3% (1-12 %)

$t_{1/2}$ optimum = 26,5h sur sulfate, 20,1 h sur thiosulfate.

Dépendante de $Na^+$

ADN: 48,6 mol% G+C (Tm)

Séquence ARN 16S: X99234

Souche type : Z-7935T (DSM 9292 T).

Origine : sédiments de lacs alcalins (lac équatorial Magadi)

Référence : Zhilina TN, Zavarzin GA, Rainey FA, Pikuta EN, Osipov GA, Kostrikina NA (1997) Desulfonatronovibrio hydrogenovorans gen. nov., sp. nov., an alkaliphilic, sulfate-reducing bacterium. Int. J. Syst. Bacteriol. 47, 144-149.

Détermination des conditions de culture optimales pour la production d'acide sulfhydrique par *Desulfonatronum lacustre*

On renvoie ici : à la figure 1a, qui représente la mesure de la densité optique au cours du temps, qui est directement reliée à la concentration des bactéries, en fonction du temps ; et à la figure 1b, qui représente la mesure de la concentration d'acide sulfhydrique dissous en fonction du temps.

Différentes conditions de culture sont testées :

F : présence de formiate ; E : présence d'éthanol ; S : présence de sulfate ; T : présence de thiosulfate ; YE : présence d'extrait de levure (Panréac, Espagne).

Les expériences ont été réalisées en double. Les figures 1a et 1b représentent la moyenne des résultats issus des mesures de la DO à 580 nm et du dosage de l'acide sulfhydrique des différentes cultures.

L'observation de ces résultats montre que :

- l'utilisation de formiate permet d'obtenir des concentrations d'acide sulfhydrique plus élevées que l'utilisation de l'éthanol ;
- les cultures en présence de thiosulfate permettent d'obtenir des concentrations d'acide sulfhydrique plus élevées qu'en présence de sulfate ;
- l'ajout d'extrait de levure dans le milieu de culture ne permet pas d'obtenir des résultats significativement différents de ceux obtenus sans cet ajout ; la raison en est que l'extrait de levure est présent à l'état de traces dans l'inoculum de bactéries ;
- *Desulfonatronum lacustre* réduit le thiosulfate par oxydation du formiate en produisant de fortes concentrations (environ 22 mM) d'acide sulfhydrique ;
- la vitesse de production de l'acide sulfhydrique est de 0,122 mM/h.

*Desulfonatronum lacustre* est donc apte à la production d'acide sulfhydrique et a été retenue pour la suite des expérimentations pour :

- son aptitude à produire de fortes concentrations d'acide sulfhydrique ;
- sa vitesse de production de l'acide sulfhydrique ;
- son alcalophilie permettant une plus grande solubilité de l'hydrogène sulfuré et une pression de sélection pour le maintien de la souche dans la culture. Les hauts pH éliminent les problèmes de contaminations exogènes.

Production d'acide sulfhydrique par *Desulfonatronum lacustre* en réacteur « bath »

1. Importance du carbonate pour la croissance

La mise en place des premières cultures de *Desulfonatronum lacustre* en réacteur batch a permis de confirmer la dépendance de cette souche vis à vis du carbonate. En effet, en absence de carbonate de sodium dans le milieu de culture, aucune croissance de cette bactérie n'a été observée pendant une semaine. L'addition d'une concentration de bicarbonate de sodium inférieure à 16,6 g/l ne permet pas non plus la croissance. Il a été supposé que le carbonate joue un rôle dans l'équilibre de dissociation du formiate par l'intermédiaire de la concentration de la phase gazeuse en $CO_2$.

2. Croissance et production à pH 9,5

Une culture de *Desulfonatronum lacustre* a été réalisée en premier lieu par ensemencement de 10 litres de milieu avec un inoculum composé de deux litres d'une culture en fin de phase exponentielle de croissance. La source d'énergie est représentée par le formiate (74 mM) et l'accepteur terminal d'électrons est le thiosulfate (20 mM). L'ajustement du pH à 9,5 est initialement réalisé avec du carbonate de sodium. La régulation du pH est ensuite réalisée par injection de soude 4N. Les résultats de l'expérience sont présentés sur la figure 2a.

La croissance de *Desulfonatronum lacustre* suit une courbe typique de forme sigmoïdale : après une phase de latence (t < 50 h), les bactéries rentrent dans une phase de croissance exponentielle (50 h < t < 200 h), puis une phase de ralentissement apparaît (t > 200 h). Une phase stationnaire est observée à partir de t = 200 h. Le formiate étant suivi en temps réel, la source d'énergie a été complétée pour une seconde série d'expérimentation à pH 9,5 dès que sa concentration a atteint 0 mM.

La densité optique de la culture est notablement faible et n'augmente que très peu lors du rajout de formiate : une limitation de la croissance semble être exercée. Le temps de génération de *Desulfonatronum lacustre* dans les conditions expérimentales est de 71,45 h à pH 9,5. Cette valeur est remarquable, même pour une bactérie thiosulfato-réductrice. Par comparaison, le temps de génération de *Desulfonatronum lacustre* dans les mêmes conditions expérimentales (milieu, formiate, thiosulfate, extrait de levure, température, pH) mais lors de sa culture en tube de Hungate (5 mL de milieu) est de 84,52 h. Il apparaît ainsi que la culture dans un fermenteur où sont maintenues agitation et régulation continue du pH et où le ratio volume liquide/volume gazeux est plus élevé permet de diminuer le temps de génération de cette bactérie.

L'observation de la consommation du formiate montre que celle-ci est corrélée à la croissance bactérienne. Peu importante lors de la phase de latence initiale, la consommation de formiate augmente lors de la phase exponentielle de croissance puis ralentit lors de la phase stationnaire. On note cependant que la consommation de formiate est maintenue, ce qui est conforme à l'observation du maintien d'une activité de production d'acide sulfhydrique par les bactéries en état stationnaire.

Par corrélation entre la quantité de formiate consommée et celle d'acide sulfhydrique produit, on déduit que dans les conditions expérimentales la formation d'une mole d'acide sulfhydrique nécessite l'oxydation de 2,83 moles de formiate à pH 9,5.

Après établissement d'une biomasse à l'intérieur du réacteur et épuisement de la source d'énergie, une corrélation a été établie entre la densité optique de la culture à 580 nm et la biomasse. Ainsi, pour une $DO_{580nm}$ de 0,465, il a été déterminé un poids sec de 67,6 mg/L. En supposant la relation biomasse = $ft(DO_{580nm})$ linéaire, on a :

$$\text{Biomasse (mg/L)} = 145,37 \times DO_{580nm}$$

La vitesse spécifique de production d'acide sulfhydrique a pu être déterminée. La vitesse spécifique de production d'acide sulfhydrique à pH 9,5 dans les conditions expérimentales est de 3,318 mmol $HS^- g^{-1} h^{-1}$.

3. Croissance et production à pH 10

La croissance de *Desulfonatronum lacustre* a ensuite été réalisée à pH 10 par ajout de milieu de culture concentré dans le réacteur et modification du point de consigne du pH. Les résultats de cette expérience sont présentés sur la figure 2b. A t = 92 h, le milieu de culture a été enrichi par ajout de 1 L milieu de culture concentré (équivalent 8 litres de milieu standard) afin de rajouter de la source d'énergie dont la concentration diminuait fortement. Cet ajout a provoqué la diminution de la concentration en acide sulfhydrique et un abaissement de la $DO_{580nm}$.

On peut observer que la croissance de *Desulfonatronum lacustre* est maintenue à pH 10. Celle-ci est toujours corrélée à l'oxydation de la source d'énergie, le formiate. Elle est cependant plus deux fois moins rapide qu'à pH 9,5. En effet le temps de génération de *Desulfonatronum lacustre* dans nos conditions expérimentales est de 141,45 h à pH 10.

On peut remarquer que la concentration d'acide sulfhydrique peut atteindre la valeur de 32 mM. Cette forte concentration de sulfure peut être à l'origine de la diminution de la vitesse de croissance de *Desulfonatronum lacustre.* Cette souche peut cependant être considérée comme exceptionnellement tolérante aux sulfures au regard des concentrations d'acide sulfhydrique rencontrées dans le milieu de culture.

Par corrélation entre la quantité de formiate consommée et celle d'acide sulfhydrique produit, nous déduisons que l'augmentation du pH du milieu de culture à une valeur de 10 ne modifie pas le rendement énergétique de la formation d'acide sulfhydrique. En effet, les valeurs à pH 9,5 et pH 10 sont presque identiques : la formation d'une mole d'acide sulfhydrique nécessite l'oxydation de 2,66 moles de formiate à pH 10.

Cependant, la vitesse spécifique de production d'acide sulfhydrique diminue légèrement (11 %) lors du passage du pH de 9,5 à 10. La vitesse spécifique de production d'acide sulfhydrique à pH 10 dans nos conditions expérimentales est de 2,974 ± 0,635 mmol $HS^- g^{-1} h^{-1}$.

Cette diminution de l'activité de *Desulfonatronum lacustre* lors du passage de pH 9,5 à pH 10 n'est pas conforme aux résultats de Pikuta et al. qui ont observé une diminution de 50% de l'activité sulfidogénique de cette souche lors du passage de pH 9,5 à pH 10.

Lors des cultures réalisées à pH 10, le milieu ajouté dans le réacteur n'a pas été stérilisé ni désaéré. Une observation microscopique a permis de contrôler que seule *Desulfonatronum lacustre* pouvait se maintenir dans ses conditions restrictives de croissance (pH élevé, milieu minéral, forte concentration de sulfures). De plus, la forte concentration de sulfures dans le réacteur permet de maintenir dans ce dernier une anoxie complète comme en témoigne le maintien d'une forte concentration d'acide sulfhydrique.

Optimisation du milieu de culture

Des cultures ont été réalisées en tube Hungate dans divers milieux de culture dérivés du milieu de culture standard MLF. Suivant la série de culture, la cystéine, le sulfure de sodium, l'acétate, l'extrait de levure ou les oligo-éléments ont été omis. La première inoculation de *Desulfonatronum lacustre* a été réalisée à partir d'une pré-culture sur milieu riche MLF. Par la suite, pour les deuxièmes (t = 10 jours) et troisièmes (t = 20 jours) repiquages, l'inoculum a été fourni par la culture réalisée précédemment sur le même type de milieu. Ces repiquages en série permettent de garantir l'absence du composé désiré par le jeu des dilutions.

Rappelons que dans le milieu MLF, la cystéine et le sulfure de sodium sont ajoutés en tant que réducteurs permettant de favoriser l'établissement d'un potentiel redox faible favorable à la croissance de *Desulfonatronum lacustre.* L'acétate est ajouté afin de fournir une source complémentaire de carbone facilement accessible à la souche bactérienne, devant lui permettre d'initier sa croissance. L'extrait de levure est une source d'acides aminés et de facteurs de croissance non organiques pour cette souche hétérotrophe et la solution des oligoéléments de Widdel apporte de nombreux métaux pouvant intervenir dans le métabolisme bactérien.

Les résultats issus du troisième repiquage des différents milieux de culture alternatifs sont représentés dans le tableau 1 ci-dessous. La comparaison de ces résultats permet de constater que les trois plus faibles croissances et les trois plus faibles productions d'acide sulfhydrique sont corrélées à l'absence d'extrait de levure (milieux B, D et I).

Une faible concentration d'extrait de levure (0,1 g/L) semble indispensable à une bonne croissance de *Desulfonatronum lacustre* et une forte production d'acide sulfhydrique par cette souche. Dans une moindre mesure, l'omission de la solution d'oligo-éléments de Widdel semble limiter la production d'acide sulfhydrique par *Desulfonatronum lacustre.* Le rôle de ces oligoéléments peut être expliqué par la présence de métaux dans les hèmes des enzymes intervenants dans la respiration du sulfate chez les bactéries sulfato-réductrices. Ces éléments pourraient être apportés par les impuretés rencontrées dans les produits de qualité industrielle et que l'on ne retrouve pas dans les produits de qualité « analyse » utilisés en laboratoire.

**TABLEAU 1**

| Composés | Concentrations (mg/l) | A | B | C | D | E | F | G | H | I |
|---|---|---|---|---|---|---|---|---|---|---|
| Cystéine | 0,5 | X | X | X | X | | X | | X | |
| Sulfure de sodium | 0,4 | X | X | X | X | X | | X | | |
| Acétate de sodium | 0,16 (2 mM) | X | X | | | X | X | | | |
| Extrait de levure | 0,1 | X | | X | | X | X | X | X | |
| Oligo-éléments de Widdel | 1 ml | | X | | X | X | X | X | X | |
| Résultats 3ème repiquage * | DO$_{580nm}$ | 0,23 | 0,18 | 0,25 | 0,18 | 0,3 | 0,22 | 0,23 | 0,24 | 0,2 |
| | HS- | 9,35 | 6,45 | 10,5 | 2,35 | 14 | 10,9 | 8,85 | 12,5 | 1,9 |

* les résultats sont la moyenne de deux séries d'expérimentations.

Précipitation du cuivre par l'acide sulfhydrique

Le dosage de l'hydrogène sulfuré dissous est réalisé par mesure de l'absorbance du sulfure de cuivre de couleur rouge bordeaux, formé par action de l'acide sulfhydrique sur le sulfate de cuivre. La réaction intervenant dans ce dosage a été mise à profit pour illustrer les capacités de l'acide sulfhydrique pour la séparation des métaux dissous dans un effluent.

Le sulfure de cuivre se forme instantanément lors du mélange sous agitation de l'acide sulfhydrique et du cuivre dissous. Le sulfure de cuivre ainsi formé précipite rapidement, de manière visible à l'oeil nu. Le mélange étant laissé au repos pendant 5 minutes, la majeure partie du précipité est retrouvée au fond de l'éprouvette.

L'ajout d'un coagulant tel que FeCl$_3$ permet de précipiter l'intégralité du cuivre.

La précipitation des métaux sous forme de sulfures par utilisation de l'acide sulfhydrique présent dans les cultures de bactéries sulfato et thiosulfato-réductrices a été confirmée.

Décontamination d'un effluent industriel

On effectue une précipitation de métaux lourds contenus dans un effluent industriel réel fourni par la société X, située dans le Vaucluse et confrontée à une contamination de ses effluents par le plomb qu'elle transforme dans le cadre de son activité de production d'accumulateurs.

Une culture de *Desulfonatronum lacustre* est réalisée à 37°C pendant plus de 2 mois dans 2 litres de milieu MLF. Le pH initial de la culture est de 9,5. La source d'énergie utilisée est le formiate, la source de soufre est le thiosulfate.

L'effluent pollué est soutiré en amont de la station de traitement actuellement existante.

Une mesure de la concentration d'acide sulfhydrique est réalisée au préalable. Après dilution au ¼ du milieu de culture, la mesure donne une concentration de $4 \times 12,9 = 51,6$ mM. Cette concentration exceptionnelle d'acide sulfhydrique dans un milieu de culture est issue d'un temps de culture particulièrement long (supérieur à 60 jours).

L'analyse du pH final du milieu montre que celui-ci est de 8,8. La chute du pH de 9,5 à 8,8 malgré la présence d'un tampon carbonate confirme la génération d'une grande quantité d'acide pendant la culture de *Desulfonatronum lacustre.*

Cette concentration d'acide sulfhydrique supérieure à 50 mM dans le milieu de culture montre que *Desulfonatronum lacustre* est capable de tolérer des concentrations particulièrement élevées de sulfures.

5 ml de milieu de culture contenant 51,6 mM d'acide sulfhydrique sont prélevés à l'aide d'une seringue et injectés dans 150 ml d'effluent contaminé. L'effluent avant injection est représenté sur la photo A de la figure 3, l'effluent immédiatement après injection est représenté sur la photo B de la figure 3.

Dès l'injection de l'acide sulfhydrique dans l'effluent, des particules de sulfures de plomb, identifiées par leur couleur caractéristique d'un noir brillant, apparaissent et sédimentent rapidement.

L'injection de milieu de culture contenant de l'acide sulfhydrique produit par le métabolisme de *Desulfonatronum lacustre* permet donc de réaliser instantanément la précipitation du plomb et sa séparation de l'effluent industriel.

**Revendications**

1.   Procédé de production d'acide sulfhydrique sous forme majoritairement soluble en milieu aqueux comportant

- une étape de culture de bactéries alcalophiles sulfato-réductrices ou thiosulfato-réductrices choisies parmi au moins une espèce de la famille des *Desulfohalobiaceae* ou du genre *Desulfonatronum* ou dont le gène codant pour l'ARN ribosomique 16 S présente une homologie d'au moins 97 % avec le gène correspondant de l'une quelconque des espèces de la famille des *Desulfohalobiaceae* ou du genre *Desulfonatronum,* en présence d'un composé organique servant de donneur d'électrons, notamment le formiate, et en présence d'un composé soufré servant d'accepteur d'électrons, notamment le thiosulfate, ce qui conduit à la formation d'acide sulfhydrique,

ledit procédé comprenant une succession des deux étapes suivantes :

- une première étape dans laquelle la culture des bactéries est effectuée dans des conditions appropriées à la croissance desdites bactéries et à la production concomitante d'acide sulfhydrique, et
- une seconde étape dans laquelle la culture des bactéries est effectuée dans des conditions appropriées à la production d'acide sulfhydrique en l'absence de croissance desdites bactéries,

la succession desdites étapes constituant un cycle pouvant éventuellement être répété plusieurs fois, et la première étape dudit procédé étant effectuée à un pH allant de 9 à 10 et la seconde étape dudit procédé étant effectuée à un pH supérieur à 10.

2.   Procédé de production d'acide sulfhydrique selon la revendication 1, dans lequel lesdites bactéries présentent une tolérance à l'acide sulfhydrique, ladite tolérance étant **caractérisée en ce que** les bactéries tolèrent des concentrations en acide sulfhydrique au moins supérieures à 20 mM.

3.   Procédé de production d'acide sulfhydrique selon l'une des revendications 1 à 2, dans lequel lesdites bactéries appartiennent à l'espèce *Desulfonatronum lacustre.*

4.   Procédé de production d'acide sulfhydrique selon l'une des revendications 1 à 2, dans lequel lesdites bactéries appartiennent à l'espèce *Desulfonatronovibrio hydrogenevorans.*

**5.** Procédé de production d'acide sulfhydrique selon l'une des revendications 3 à 4, dans lequel ladite culture est effectuée sur un support adapté à la croissance des bactéries, conduisant à la formation d'un biofilm.

**6.** Procédé de production d'acide sulfhydrique selon la revendication 5, dans lequel le passage d'une étape à l'autre est effectué notamment au moyen d'ajout d'une ou plusieurs substances chimiques acides ou basiques entraînant une modification du pH du milieu de culture des bactéries.

**7.** Procédé de production d'acide sulfhydrique selon l'une des revendications 1 à 6, dans lequel l'acide sulfhydrique est produit à une concentration supérieure ou égale à 10 mM, notamment à une concentration supérieure ou égale à 20 mM, notamment à une concentration supérieure ou égale à 30 mM.

**8.** Procédé de production d'acide sulfhydrique selon l'une des revendications 1 à 6, dans lequel lesdites bactéries appartiennent à l'espèce *Desulfonatronum lacustre,* le composé organique servant de donneur d'électrons est le formiate, le composé soufré servant d'accepteur d'électrons est le thiosulfate, les bactéries sont cultivées en continu sur un biofilm

**9.** Procédé de dépollution d'un effluent contenant un ou plusieurs métaux solubilisés comportant :

- une étape de production d'acide sulfhydrique sous forme majoritairement soluble en milieu aqueux au moyen d'une culture de bactéries alcalophiles sulfato-réductrices ou thiosulfato-réductrices choisies parmi au moins une espèce de la famille des *Desulfohalobiaceae* ou du genre *Desulfonatronum* ou dont le gène codant pour l'ARN ribosomique 16 S présente une homologie d'au moins 97 % avec le gène correspondant de l'une quelconque des espèces de la famille des *Desulfohalobiaceae* ou du genre *Desulfonatronum* en présence d'un composé organique servant de donneur d'électrons, notamment le formiate, et en présence d'un composé soufré servant d'accepteur d'électrons, notamment le thiosulfate, et
- une étape de mise en contact dudit effluent avec l'acide sulfhydrique obtenu à l'étape précédente, entraînant la réduction desdits métaux solubilisés et / ou la précipitation desdits métaux solubilisés sous forme de sulfures métalliques, ladite mise en contact s'effectuant à un pH allant de 2 à 12,

l'étape de production d'acide sulfhydrique comprenant une succession des deux étapes suivantes :

- une première étape dans laquelle la culture des bactéries est effectuée dans des conditions appropriées à la croissance desdites bactéries et à la production concomitante d'acide sulfhydrique, et
- une seconde étape dans laquelle la culture des bactéries est effectuée dans des conditions appropriées à la production d'acide sulfhydrique en l'absence de croissance desdites bactéries,

la succession desdites étapes constituant un cycle pouvant éventuellement être répété plusieurs fois, et la première étape dudit procédé étant effectuée à un pH allant de 9 à 10 et la seconde étape dudit procédé étant effectuée à un pH supérieur à 10.

**10.** Procédé de dépollution d'un effluent contenant un ou plusieurs métaux solubilisés selon la revendication 9, dans lequel l'étape de production d'acide sulfhydrique est effectuée selon le procédé de l'une quelconque des revendications 1 à 8.

**11.** Procédé de dépollution d'un effluent contenant un ou plusieurs métaux solubilisés selon la revendication 9 ou 10, dans lequel l'étape de mise en contact de l'effluent avec l'acide sulfhydrique est effectuée à un pH neutre ou basique.

**12.** Procédé de dépollution d'un effluent contenant un ou plusieurs métaux solubilisés selon la revendication 10 ou 11, dans lequel on effectue la production d'acide sulfhydrique et la mise en contact de l'effluent avec l'acide sulfhydrique dans des cuves distinctes, notamment respectivement une cuve de culture et une cuve de réaction, ledit procédé comportant une étape intermédiaire entre l'étape de production d'acide sulfhydrique et l'étape de mise en contact de l'effluent avec l'acide sulfhydrique, ladite étape intermédiaire consistant en l'injection de tout ou partie de l'acide sulfhydrique produit dans la cuve de culture dans la cuve de réaction.

**13.** Procédé de dépollution d'un effluent contenant un ou plusieurs métaux solubilisés selon la revendication 12, dans lequel aucune fraction de l'effluent n'est injectée dans la cuve de culture.

**14.** Procédé de dépollution d'un effluent contenant un ou plusieurs métaux solubilisés selon la revendication 12, dans lequel une fraction de l'effluent est injectée dans la cuve de culture.

**15.** Procédé de dépollution d'un effluent contenant un ou plusieurs métaux solubilisés selon l'une des revendications 12 à 14, dans lequel l'étape de mise en contact de l'effluent avec l'acide sulfhydrique dans la cuve de réaction est effectuée en absence d'une phase gazeuse, de telle sorte qu'il n'y a pas de dégagement de sulfure d'hydrogène gazeux.

**16.** Procédé de dépollution d'un effluent contenant un ou plusieurs métaux solubilisés selon l'une des revendications 12 à 15 comportant les étapes supplémentaires :

- de mesure de la concentration d'acide sulfhydrique produit dans la cuve de culture,

- d'estimation de la concentration du ou des différents métaux solubilisés dans l'effluent devant être dépollué, et

- d'ajustement de la quantité de solution contenant de l'acide sulfhydrique devant être injectée dans la cuve de réaction en fonction du résultat de ladite mesure et de ladite estimation.

**17.** Procédé de dépollution d'un effluent contenant un ou plusieurs métaux solubilisés selon l'une des revendications 9 à 16, dans lequel le métal ou les métaux solubilisés dans l'effluent pollué est choisi ou sont choisis parmi le cuivre, le zinc, l'arsenic, le cadmium, le chrome, notamment sous sa forme hexavalente, l'étain, le manganèse, le mercure, le nickel, et le plomb.


**Patentansprüche**

**1.** Verfahren zur Herstellung von Wasserstoffsulfid in einer in wässrigem Medium überwiegend löslichen Form, umfassend

- einen Schritt zum Kultivieren von alkalophilen, sulfatreduzierenden oder thiosulfatreduzierenden Bakterien, ausgewählt aus mindestens einer Spezies der Familie der *Desulfohalobiaceae* oder der Gattung *Desulfonatronum* oder deren Gen, das für die ribosomale 16S-RNA kodiert, eine Homologie von mindestens 97 % mit dem Gen aufweist, das irgendeiner der Spezies der Familie der *Desulfohalobiaceae* oder der Gattung *Desulfonatronum* entspricht, in Gegenwart einer organischen Verbindung, die als Elektronenspender dient, insbesondere Formiat, und in Gegenwart einer Schwefelverbindung, die als Elektronenakzeptor dient, insbesondere Thiosulfat, was zur Bildung von Wasserstoffsulfid führt,

wobei das Verfahren die Abfolge der beiden folgenden Schritte umfasst:

- einen ersten Schritt, wobei das Kultivieren der Bakterien unter Bedingungen durchgeführt wird, die sowohl für das Wachstum der Bakterien als auch für die gleichzeitige Herstellung von Wasserstoffsulfid geeignet sind, und
- einen zweiten Schritt, wobei das Kultivieren der Bakterien unter Bedingungen durchgeführt wird, die für die Herstellung von Wasserstoffsulfid in Abwesenheit von Wachstum der Bakterien geeignet sind,

wobei die Abfolge der Schritte einen Zyklus bildet, der gegebenenfalls mehrmals wiederholt werden kann, und wobei der erste Schritt des Verfahrens bei einem pH-Wert durchgeführt wird, der zwischen 9 und 10 liegt, und der zweite Schritt des Verfahrens bei einem pH-Wert durchgeführt wird, der größer als 10 ist.

**2.** Verfahren zur Herstellung von Wasserstoffsulfid nach Anspruch 1, wobei die Bakterien eine Toleranz gegen Wasserstoffsulfid aufweisen, wobei die Toleranz **dadurch gekennzeichnet ist, dass** die Bakterien Wasserstoffsulfidkonzentrationen von mindestens größer als 20 mM tolerieren.

**3.** Verfahren zur Herstellung von Wasserstoffsulfid nach einem der Ansprüche 1 bis 2, wobei die Bakterien zur Spezies *Desulfonatronum lacustre* gehören.

**4.** Verfahren zur Herstellung von Wasserstoffsulfid nach einem der-Ansprüche 1 bis 2, wobei die Bakterien zur Spezies *Desulfonatronovibrio hydrogenevorans* gehören.

**5.** Verfahren zur Herstellung von Wasserstoffsulfid nach einem der Ansprüche 3 bis 4, wobei das Kultivieren auf einem Träger durchgeführt wird, der für das Wachstum der Bakterien geeignet ist, was zur Bildung eines Biofilms führt.

6.  Verfahren zur Herstellung von Wasserstoffsulfid nach Anspruch 5, wobei der Übergang von einem Schritt zum nächsten insbesondere mithilfe der Zugabe einer oder mehrerer saurer oder basischer chemischer Substanzen durchgeführt wird, die zu einer Änderung des pH-Werts des Kulturmediums der Bakterien mit sich bringt.

7.  Verfahren zur Herstellung eines Wasserstoffsulfidsnach einem der Ansprüche 1 bis 6, wobei das Wasserstoffsulfid mit einer Konzentration größer oder gleich 10 mM, insbesondere einer Konzentration größer oder gleich 20 mM, insbesondere mit einer Konzentration größer oder gleich 30 mM hergestellt wird.

8.  Verfahren zur Herstellung von Wasserstoffsulfid nach einem der Ansprüche 1 bis 6, wobei die Bakterien zur Spezies *Desulfonatronum lacustre* gehören, die organische Verbindung, die als Elektronendonor dient, Formiat ist, die Schwefelverbindung, die als Elektronenakzeptor dient, Thiosulfat ist, die Bakterien kontinuierlich auf einem Biofilm kultiviert werden.

9.  Verfahren zur Entgiftung eines Abflusses, der ein oder mehrere solubilisierte Metalle enthält, umfassend:

    - einen Schritt zur Herstellung von Wasserstoffsulfid in einer in wässrigem Medium überwiegend löslichen Form mithilfe einer Kultur von alkalophilen, sulfatreduzierenden oder thiosulfatreduzierenden Bakterien, ausgewählt aus mindestens einer Spezies der Familie der *Desulfohalobiaceae* oder der Gattung *Desulfonatronum* oder deren Gen, das für die ribosomale 16S-RNA kodiert, eine Homologie von mindestens 97 % mit dem Gen aufweist, das irgendeiner der Spezies der Familie der *Desulfohalobiaceae* oder der Gattung *Desulfonatronum* entspricht, in Gegenwart einer organischen Verbindung, die als Elektronenspender dient, insbesondere Formiat, und in Gegenwart einer Schwefelverbindung, die als Elektronenakzeptor dient, insbesondere Thiosulfat, und
    - einen Schritt zum Inkontaktbringen des Abflusses mit dem Wasserstoffsulfid, das im vorhergehenden Schritt erhalten wurde, der zur Reduktion der solubilisierten Metalle und/oder dem Ausfällen der solubilisierten Metalle in Form von Metallsulfiden führt, wobei das Inkontaktbringen bei einem pH-Wert zwischen 2 und 12 erfolgt,

    wobei der Schritt zum Herstellen von Wasserstoffsulfid eine Abfolge der beiden folgenden Schritte umfasst:

    - einen ersten Schritt, wobei das Kultivieren der Bakterien unter Bedingungen durchgeführt wird, die sowohl für das Wachstum der Bakterien als auch für die gleichzeitige Herstellung von Wasserstoffsulfid geeignet sind, und
    - einen zweiten Schritt, wobei das Kultivieren der Bakterien unter Bedingungen durchgeführt wird, die für die Herstellung von Wasserstoffsulfid in Abwesenheit von Wachstum der Bakterien geeignet sind,

    wobei die Abfolge der Schritte einen Zyklus bildet, der gegebenenfalls mehrmals wiederholt werden kann, und wobei der erste Schritt des Verfahrens bei einem pH-Wert durchgeführt wird, der zwischen 9 und 10 liegt, und der zweite Schritt des Verfahrens bei einem pH-Wert durchgeführt wird, der größer als 10 ist.

10. Verfahren zur Entgiftung eines Abflusses, der ein oder mehrere solubilisierte Metalle nach Anspruch 9 enthält, wobei der Schritt zur Herstellung von Wasserstoffsulfid gemäß dem Verfahren nach einem der Ansprüche 1 bis 8 durchgeführt wird.

11. Verfahren zur Entgiftung eines Abflusses, der ein oder mehrere solubilisierte Metalle nach Anspruch 9 oder 10 enthält, wobei der Schritt zum Inkontaktbringen des Abflusses mit Wasserstoffsulfid bei einem neutralen oder basischen pH-Wert durchgeführt wird.

12. Verfahren zur Entgiftung eines Abflusses, der ein oder mehrere solubilisierte Metalle nach Anspruch 10 oder 11 enthält, wobei die Herstellung von Wasserstoffsulfid und das Inkontaktbringen des Abflusses mit dem Wasserstoffsulfid in verschiedenen Behältern durchgeführt wird, insbesondere jeweils einem Kulturbehälter und einem Reaktionsbehälter, wobei das Verfahren zwischen dem Schritt zur Herstellung von Wasserstoffsulfid und dem Schritt zum Inkontaktbringen des Abflusses mit Wasserstoffsulfid einen Zwischenschritt umfasst, wobei der Zwischenschritt in der Injektion des gesamten oder eines Teils des Wasserstoffsulfids, das in dem Kulturbehälter hergestellt wurde, in den Reaktionsbehälter besteht.

13. Verfahren zur Entgiftung eines Abflusses, der ein oder mehrere solubilisierte Metalle nach Anspruch 12 enthält, wobei keine Fraktion des Abflusses in den Kulturbehälter injiziert wird.

**14.** Verfahren zur Entgiftung eines Abflusses, der ein oder mehrere solubilisierte Metalle nach Anspruch 12 enthält, wobei eine Fraktion des Abflusses in den Kulturbehälter injiziert wird.

**15.** Verfahren zur Entgiftung eines Abflusses, der ein oder mehrere solubilisierte Metalle nach einem der Ansprüche 12 bis 14 enthält, wobei der Schritt zum Inkontaktbringen des Abflusses mit dem Wasserstoffsulfid in dem Reaktionsbehälter in Abwesenheit eine Gasphase derart durchgeführt wird, dass keine Freisetzung von gasförmigem Schwefelwasserstoff stattfindet.

**16.** Verfahren zur Entgiftung eines Abflusses, der ein oder mehrere solubilisierte Metalle nach einem der Ansprüche 12 bis 15 enthält, das die zusätzlichen Schritte:

   - zum Messen der Konzentration des Wasserstoffsulfids, das in dem Kulturbehälter hergestellt wurde,
   - zum Schätzen der Konzentration des oder der verschiedenen Metalle, die in dem Abfluss solubilisiert sind, bevor er entgiftet wird, und
   - zum Anpassen der Menge an Wasserstoffsulfid enthaltender Lösung, die in den Reaktionsbehälter injiziert werden soll, in Abhängigkeit vom Ergebnis der Messung und der Schätzung umfasst.

**17.** Verfahren zum Entgiften eines Abflusses, der ein oder mehrere solubilisierte Metalle nach einem der Ansprüche 9 bis 16 enthält, wobei das Metall oder die Metalle, das bzw. die in dem verschmutzten Abfluss solubilisiert ist bzw. sind, ausgewählt sind aus Kupfer, Zink, Arsen, Cadmium, Chrom, insbesondere in hexavalenter Form, Zinn, Mangan, Quecksilber, Nickel, und Blei.

## Claims

**1.** Process for the production of hydrogen sulphide in a substantially soluble form in an aqueous medium comprising

   - a step of culturing alkaliphilic sulphate-reducing or thiosulphate-reducing bacteria chosen among at least one species of the *Desulfohalobiaceae* family or of the *Desulfonatronum* genus or at least one species of which the gene coding for the ribosomal RNA 16 S exhibits a homology of at least 97 % with the corresponding gene of any one of the species of the *Desulfohalobiaceae* family or of the *Desulfonatronum* genus, in the presence of an organic compound used as an electron donor, in particular formate, and in the presence of a sulphurous compound used as an electron acceptor, in particular thiosulphate, which leads to the formation of hydrogen sulphide,

said process comprising a succession of the following two steps:

   - a first step wherein the culture of bacteria is carried out under conditions appropriate for the growth of said bacteria and for the concomitant production of hydrogen sulphide, and
   - a second step wherein the culture of bacteria is carried out under appropriate conditions for the production of hydrogen sulphide in the absence of growth of said bacteria,

the succession of said steps constituting a cycle which can be possibly repeated several times, and
the first step of said process being carried out at a pH ranging from 9 to 10 and the second step of said process being carried out at a pH greater than 10.

**2.** Process for the production of hydrogen sulphide according to claim 1, wherein said bacteria have a tolerance to hydrogen sulphide, said tolerance being **characterised in that** the bacteria tolerate concentrations of hydrogen sulphide at least greater than 20 mM.

**3.** Process for the production of hydrogen sulphide according to any one of claims 1 to 2, wherein said bacteria belong to the *Desulfonatronum lacustre* species.

**4.** Process for the production of hydrogen sulphide according to any one of claims 1 to 2, wherein said bacteria belong to the *Desulfonatronovibrio hydrogenevorans* species.

**5.** Process for the production of hydrogen sulphide according to any one of claims 3 to 4, wherein said culture is carried out on a support suitable for the growth of bacteria, leading to the formation of a biofilm.

6.  Process for the production of hydrogen sulphide according to claim 5, wherein the passage from one step to the other is in particular carried out by means of addition of one or several acidic or basic chemical substances leading to a pH modification of the culture medium of bacteria.

7.  Process for the production of hydrogen sulphide according to any one of claims 1 to 6, wherein the hydrogen sulphide is produced at a concentration greater than or equal to 10 mM, in particular at a concentration greater than or equal to 20 mM, in particular at a concentration greater than or equal to 30 mM.

8.  Process for the production of hydrogen sulphide according to any one of claims 1 to 6, wherein said bacteria belong to the *Desulfonatronum lacustre* species, the organic compound being used as an electron donor is formate, the sulphurous compound used as an electron acceptor is thiosulphate, the bacteria are cultured in continuous on a biofilm.

9.  Process for the decontamination of an effluent containing one or several dissolved metals comprising:

    - a step of production of hydrogen sulphide in a substantially soluble form in an aqueous medium by means of a culture of alkaliphilic sulphate-reducing or thiosulphate-reducing bacteria chosen among from at least one species of the *Desulfohalobiaceae* family or of the *Desulfonatronum* genus or at least one species of which the gene coding for the ribosomal RNA 16 S exhibits a homology of at least 97 % with the corresponding gene of any one of the species of the *Desulfohalobiaceae* family or of the *Desulfonatronum* genus in the presence of an organic compound used as an electron donor, in particular formate, and in the presence of a sulphurous compound used as an electron acceptor, in particular thiosulphate, and
    - a step of contacting said effluent with the hydrogen sulphide obtained in the preceding step, leading to the reduction of said dissolved metals and/or the precipitation of said dissolved metals in the form of metal sulphides, said contacting being carried out at a pH ranging from 2 to 12,

    the step of production of hydrogen sulphide comprising a succession of the following two steps:

    - a first step wherein the culture of bacteria is carried out under appropriate conditions for the growth of said bacteria and for the concomitant production of hydrogen sulphide, and
    - a second step wherein the culture of bacteria is carried out under appropriate conditions for the production of hydrogen sulphide in the absence of growth of said bacteria,

    the succession of said steps constituting a cycle which can be possibly repeated several times, and the first step of said process being carried out at a pH ranging from 9 to 10 and the second step of said process being carried out at a pH greater than 10.

10. Process for the decontamination of an effluent containing one or several dissolved metals according to claim 9, wherein the step of production of hydrogen sulphide is carried out according to the process of any one of claims 1 to 8.

11. Process for the decontamination of an effluent containing one or several dissolved metals according to claim 9 or 10, wherein the step of contacting the effluent with the hydrogen sulphide is carried out at a neutral or basic pH.

12. Process for the decontamination of an effluent containing one or several dissolved metals according to claim 10 or 11, wherein the production of hydrogen sulphide and the contacting of the effluent with the hydrogen sulphide are carried out in separate tanks, in particular respectively a culture tank and a reaction tank, said process comprising an intermediate step between the hydrogen sulphide production step and the step of contacting the effluent with the hydrogen sulphide, said intermediate step consisting in the injection of all or part of the hydrogen sulphide produced in the culture tank into the reaction tank.

13. Process for the decontamination of an effluent containing one or several dissolved metals according to claim 12, wherein no fraction of the effluent is injected into the culture tank.

14. Process for the decontamination of an effluent containing one or several dissolved metals according to claim 12, wherein a fraction of the effluent is injected into the culture tank.

15. Process for the decontamination of an effluent containing one or several dissolved metals according to one of claims 12 to 14, wherein the step of contacting the effluent with the hydrogen sulphide in the reaction tank is carried out in the absence of a gaseous phase, in such a manner that there is no release of gaseous hydrogen sulphide.

16. Process for the decontamination of an effluent containing one or more dissolved metals according to one of claims 12 to 15 comprising the supplementary steps of:

- measuring the concentration of hydrogen sulphide produced in the culture tank,
- estimating the concentration of the metal or different metals dissolved in the effluent that have to be decontaminated, and
- adjusting the quantity of solution containing hydrogen sulphide that has to be injected into the reaction tank depending on the result of said measurement and of said estimation.

17. Process for the decontamination of an effluent containing one or more dissolved metals according to one of claims 9 to 16, wherein the metal or metals dissolved in the polluted effluent is or are chosen from copper, zinc, arsenic, cadmium, chromium, particularly in its hexavalent form, tin, manganese, mercury, nickel, and lead.

FIGURE 1a

EP 1 874 941 B1

FIGURE 1b

FIGURE 2a

FIGURE 2b

A

B

FIGURE 3

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 8002281 A
- US 4522723 A
- US 4108722 A
- US 5062956 A
- US 5587079 A
- WO 9729055 A
- WO 0206540 A
- WO 9705237 A
- US 4789478 A
- EP 0692458 A

**Littérature non-brevet citée dans la description**

- **PIKUTA et al.** Desulfonatronum lacustre gen. nov. sp. nov.: a new alkaliphilic sulfate-reducing bacterium utilizing ethanol. *Microbiology,* vol. 67, 105
- **ZHILINA et al.** Desulfonatronovibrio hydrogenovorans gen. nov. sp. nov., an alkaliphilic, sulfate-reducing bacterium. *Int. J. Syst. Bacteriol.,* Janvier 1997, 144
- **PIKUTA et al.** Desulfonatronum thiodismutans sp. nov., a novel alkaliphilic, sulfate-reducing bacterium capable of lithoautotrophic growth. *Int. J. Syst. Evol. Microbiol.,* vol. 53, 1327
- **ALTSCHUL et al.** *Nucl. Acid Res.,* 1997, vol. 25, 3389
- **THOMPSON et al.** *Nucl. Acid Res.,* 1994, vol. 22, 4673
- **KIM et al.** Chromium VI reduction by hydrogen sulfide in aqueous media: stoichiometry and kinetics. *Environ. Sci. Technol.,* 2001, vol. 35 (11), 2219-2225
- **CORD RUWISH R.** *J. Microbiol. Methods.,* 1985, vol. 4, 33-36
- **NOR Y.M ; TABATABAI M.A.** *Anal. Lett.,* 1975, vol. 8, 537-547
- **TABATABAI M.A.** *Sulfur Institut Journal,* 1974, vol. 10, 11-13
- **PIKUTA EV ; ZHILINA TN ; ZAVARZIN GA ; KOSTRIKINA NA ; OSIPOV GA ; RAINEY FA.** Desulfonatronum lacustre gen. nov., sp. nov., a new alkaliphilic sulfate-reducing bacterium utilizing ethanol. *Microbiology (Engl. Tr. Mikrobiologyia),* 1998, vol. 67, 123-131
- **ZHILINA TN ; ZAVARZIN GA ; RAINEY FA ; PIKUTA EN ; OSIPOV GA ; KOSTRIKINA NA.** Desulfonatronovibrio hydrogenovorans gen. nov., sp. nov., an alkaliphilic, sulfate-reducing bacterium. *Int. J. Syst. Bacteriol.,* 1997, vol. 47, 144-149